# EUROPEAN PATENT APPLICATION

(11) **EP 3 666 181 A1**
(43) Date of publication of application: **17.06.2020**
(21) Application number: 19214705.6
(22) Date of filing: 10.12.2019
(51) Int. Cl.: A61B 5/044, A61B 5/0452

(54) **DISPLAY OF ARRHYTHMIA TYPE**

(30) Priority: 11.12.2018 US 201862778022 P; 28.08.2019 US 201916553745
(71) Applicant: Biosense Webster (Israel) Ltd., Yokneam, 2066717 (IL)
(72) Inventor: ZIV-ARI, Morris, 2066717 Yokneam (IL); BENAROYA, Yoav, 2066717 Yokneam (IL); STEINBERG, Dan, 2066717 Yokneam (IL); KANETTI, Adi, 2066717 Yokneam (IL); ILAN, Ido, 2066717 Yokneam (IL)
(74) Representative: Small, Gary James

(57) **Abstract**

One embodiment includes a medical system including electrodes configured for application to a body of a subject and configured to output signals in response to electrical activity of a heart of the subject, a display, and a processor configured to store a set of templates corresponding to different types of arrhythmia of the heart, apply the templates to the signals so as to associate one or more different temporal sections of the signals with respective ones of the types of arrhythmia, and render to the display a user interface screen including at least a part of the signals and graphical identifiers identifying the different temporal sections of at least the part of the signals with the respective ones of the types of arrhythmia with which they are associated.

## Description

### RELATED APPLICATION INFORMATION

This application claims the benefit of United States US Provisional Application No. 62/778,022 filed on December 11, 2018.

### FIELD OF THE INVENTION

The present invention generally relates to signal analysis, and specifically to analysis of signals generated during a medical procedure.

### BACKGROUND

The process of electrocardiography (ECG) records electrical activity of the heart over a time period using electrodes applied to the skin of a living subject. The electrodes detect electrical charges on the skin that arise from the heart's electrophysiologic pattern of depolarizing and repolarizing during each heartbeat.

Electrocardiography may be performed to detect cardiac problems such as arrhythmia of the heart as well as to monitor improvement in the condition of the heart after corrective procedures, for example, but not limited to, ablation therapy.

A standard electrocardiograph may include connections to ten electrodes which are applied to the skin of the living subject including on the subject's limbs and on the chest. The electrical potential of the heart is then measured using the ten electrodes and is recorded over a time period. In this manner, the overall magnitude and direction of the heart's electrical depolarization is captured at each moment throughout the cardiac cycle. A graph of voltage versus time may be produced yielding an electrocardiogram.

During a medical procedure such as cardiac ablation, there are typically simultaneous streams of real-time data that an operator (e.g., a physician) monitors while performing the procedure. For example, while using an intracardiac catheter to perform an ablation on intracardiac tissue, the operator may want to keep track of real-time electrophysiological (EP) data such as ECG data and ancillary data such as locations of the catheter's distal tip and ablation energy being delivered to the heart tissue.

US Patent No. 8,700,136 to Rubinstein describes a method for analyzing signals, including sensing a time-varying intracardiac potential signal and finding a fit of the time-varying intracardiac potential signal to a predefined oscillating waveform. The method further includes estimating an annotation time of the signal responsive to the fit.

US Patent Publication No. 2011/0238127 of Conley, et al., describes systems, devices, structures, and methods to present a visual display based on data from an implantable medical device. The display includes a chart showing the frequency of a detected type of arrhythmia over a predetermined period of time.

US Patent Publication No. 2010/0280400 of Ettori, et al., describes a cardiac rhythm management system used to detect episode beats associated with cardiac events in a subject's body. These events may be monitored and depolarization morphology information can be derived for candidate arrhythmic beats in an arrhythmia episode. An arrhythmic beat morphology template may be formed from selecting at least one of the candidate arrhythmic beats based upon user's labeling according to specific morphologies of one or more candidate episodes.

US Patent Publication No. 2009/0005826 of Li describes an implantable cardioverter/defibrillator (ICD) including a tachyarrhythmia detection and classification system that classifies tachyarrhythmias based on a morphological analysis of arrhythmic waveforms and a template waveform. Correlation coefficients each computed between morphological features of an arrhythmic waveform and morphological features of the template waveform provide for the basis for classifying the tachyarrhythmia. In one embodiment, a correlation analysis takes into account the uncertainty associated with the production of the template waveform by using a template band that includes confidence intervals.

US Patent No. 4,336,810 to Anderson, et al., describes a method and apparatus for arrhythmia analysis of Holter-type ECG recordings including a tape playback unit having an analog signal output of successive ECG complexes or heart beats, a converter for generating data composites representing each complex, a computer for making and storing data templates in bins corresponding to operator classification of complexes as normal, supraventricular ectopic (SVE), ventricular ectopic (VE), or unknown, for comparing successive composites with stored templates and conditions to determine a match condition, and for signaling a corresponding event to an event counter and display. In learn mode the computer signals the tape playback to stop and to display an unmatched complex for operator classification and possible subsequent generation of a new template, after which scanning is resumed.

US Patent Publication No. 2001/0056245 of Mlynash, et al., describes a noninvasive localization, characterization and classification apparatus and method for cardiac arrhythmias that enables discrete isolation of the intricate spatial and temporal detail in morphology of the atrial activity of interest from superimposed ventricular activity of a preceding heartbeat in a particular arrhythmia. An adaptive QRST subtraction template is used that is modulated for discrepancies in voltage and rate between the QRST template and arrhythmia signal. The QRST template modulation is accomplished by using one or more fiducial points and windows that are annotated in both the QRST template and the arrhythmia signal.

### SUMMARY

There is provided in accordance with an embodiment of the present invention, a medical system including electrodes configured for application to a body of a subject and configured to output signals in response to electrical activity of a heart of the subject, a display, and a processor configured to store a set of templates corresponding to different types of arrhythmia of the heart, apply the templates to the signals so as to associate one or more different temporal sections of the signals with respective ones of the types of arrhythmia, and render to the display a user interface screen including at least a part of the signals and graphical identifiers identifying the different temporal sections of at least the part of the signals with the respective ones of the types of arrhythmia with which they are associated.

Further in accordance with an embodiment of the present invention, the graphical identifiers are color-coded identifiers using a different color for different ones of the types of arrhythmia.

Still further in accordance with an embodiment of the present invention, the graphical identifiers are disposed in boxes below or above at least the part of the signals.

Additionally, in accordance with an embodiment of the present invention, the processor is configured to compute a relative proportion of the types of arrhythmia associated with the signals and render to the display the user interface screen including at least the part of the signals, the graphical identifiers, and statistics indicating the relative proportion of the types of arrhythmia associated with the signals.

Moreover, in accordance with an embodiment of the present invention, the statistics are disposed in a color-coded legend having a plurality of legend keys, each legend key textually describing one of the types of arrhythmia corresponding with one of the color-coded identifiers.

Further in accordance with an embodiment of the present invention, the processor is configured to compute a relative proportion of the types of arrhythmia associated with the signals and render to the display the user interface screen including at least the part of the signals, the graphical identifiers, and statistics indicating the relative proportion of the types of arrhythmia associated with the signals.

Still further in accordance with an embodiment of the present invention, the processor is configured to render to the display another user interface screen including at least a part of another signal describing variations in a factor used in the analysis of the heart, and graphical identifiers identifying different sections of at least the part of the other signal with different ranges of the variations in the factor, the factor being selected from any one of the following: a force applied by a probe, a tissue proximity to the probe, a velocity of the probe, a cycle length, a position stability of the probe, and a local activation time (LAT) stability.

Additionally, in accordance with an embodiment of the present invention, the processor is configured to generate the other user interface screen also to include statistics indicating a relative proportion of the different ranges of the variations in the factor.

There is also provided in accordance with still another embodiment of the present invention, a medical method including receiving signals output by electrodes applied to a body of a subject in response to electrical activity of a heart of the subject, storing a set of templates corresponding to different types of arrhythmia of the heart, applying the templates to the signals so as to associate one or more different temporal sections of the signals with respective ones of the types of arrhythmia, and rendering to a display a user interface screen including at least a part of the signals and graphical identifiers identifying the different temporal sections of at least the part of the signals with the respective ones of the types of arrhythmia with which they are associated.

Moreover, in accordance with an embodiment of the present invention, the graphical identifiers are color-coded identifiers using a different color for different ones of the types of arrhythmia.

Further in accordance with an embodiment of the present invention, the graphical identifiers are disposed in boxes below or above at least the part of the signals.

Still further in accordance with an embodiment of the present invention, the method includes computing a relative proportion of the types of arrhythmia associated with the signals, wherein the rendering includes rendering to the display the user interface screen including at least the part of the signals, the graphical identifiers, and statistics indicating the relative proportion of the types of arrhythmia associated with the signals.

Additionally, in accordance with an embodiment of the present invention, the statistics are disposed in a color-coded legend having a plurality of legend keys, each legend key textually describing one of the types of arrhythmia corresponding with one of the color-coded identifiers.

Moreover, in accordance with an embodiment of the present invention, the method includes computing a relative proportion of the types of arrhythmia associated with the signals, wherein the rendering includes rendering to the display the user interface screen including at least the part of the signals, the graphical identifiers, and statistics indicating the relative proportion of the types of arrhythmia associated with the signals.

Further in accordance with an embodiment of the present invention, the method includes rendering to the display another user interface screen including at least a part of another signal describing variations in a factor used in analysis of the heart, and graphical identifiers identifying different sections of at least the part of the other signal with different ranges of the variations in the factor, the factor being selected from any one of the following a force applied by a probe, a tissue proximity to the probe, a velocity of the probe, a cycle length, a position stability of the probe, and a local activation time (LAT) stability.

Still further in accordance with an embodiment of the present invention, the method includes generating the other user interface screen also to include statistics indicating a relative proportion of the different ranges of the variations in the factor.

There is also provided in accordance with still another embodiment of the present invention a software product, including a non-transient computer-readable medium in which program instructions are stored, which instructions, when read by a central processing unit (CPU), cause the CPU to receive signals output by electrodes applied to a body of a subject in response to electrical activity of a heart of the subject, store a set of templates corresponding to different types of arrhythmia of the heart, apply the templates to the signals so as to associate one or more different temporal sections of the signals with respective ones of the types of arrhythmia, and render to a display a user interface screen including at least a part of the signals and graphical identifiers identifying the different temporal sections of at least the part of the signals with the respective ones of the types of arrhythmia with which they are associated.

### BRIEF DESCRIPTION OF THE DRAWINGS

The foregoing and other features and advantages of the invention will be apparent from the following, more particular description of preferred embodiments of the invention, as illustrated in the accompanying drawings.
Fig. 1 a schematic illustration of an invasive medical procedure using an apparatus according to an exemplary embodiment of the present invention.
Fig. 2 is a schematic illustration of a distal end of a probe used in the apparatus of Fig. 1 according to an exemplary embodiment of the present invention.
Fig. 3 is a view of an exemplary user interface screen rendered by the apparatus of Fig. 1.
Fig. 4 is a view of a flowchart including exemplary steps in a method of operation of the apparatus of Fig. 1.

### DESCRIPTION OF EXAMPLE EMBODIMENTS

### OVERVIEW

In analyzing ECG signals (body surface or intra-cardiac) for different types of arrhythmia, a physician may visually inspect graphs of the signals, and decide from the inspection what type and when the arrhythmia occurred. This sort of visual inspection is time-consuming, and is also open to error. Correct identification of arrhythmias is particularly critical during cardiac procedures such as ablation therapy, wherein ablations may be targeted according to the different arrhythmias present.

In some systems, due to the large amount of data produced during a procedure, for example, ablation and ECG data, the physician may set different filters to filter data according to what the physician deems to be the most important. For example, although the ECG data may be indicative of many different arrhythmias, the physician may set filters to detect one or more of the different types of arrhythmia based on pattern matching. As the heart rhythm may change while the procedure continues, for example, due to corrective measures being performed, the physician may change the filters during the procedure. Although employing the pattern matching reduces error, having to make an educated guess which filters are appropriate is still time-consuming and is also open to error.

In exemplary embodiments of the present invention, an apparatus renders a user interface screen to a display showing different temporal sections of ECG signals being automatically identified with respective types of arrhythmia. The different temporal sections may be identified with the respective types of arrhythmia using different colored and/or patterned identifiers and/or any suitable format including letters and/or digits.

Automatically identifying the different arrhythmias in the ECG signals reduces error and time spent associated with manual inspections. Additionally, as the ECG signals include the different types of arrhythmia, the physician can see at the same time all the arrhythmias included in the captured ECG signals shown in the user interface screen. This is particularly advantageous during a cardiac procedure where the flow of the procedure may be guided by the various arrhythmias that may need to be treated.

The user interface screen may also include statistics indicating the relative proportion of the types of arrhythmia associated with the signals. This may easily enable the physician to prioritize the cardiac procedure according to the most prominent arrhythmias identified in the ECG data.

The signals and its identifiers may be scrolled (left and right) to show different temporal sections of the signals.

The data included in the user interface screen may be updated as the procedure progresses showing the relative proportion of the remaining types of arrhythmia in the ECG data thereby providing the physician with an indication of the current success of the procedure as well as the importance of any remaining arrhythmias.

Additionally, or alternatively, the apparatus may filter historical ECG data from any prior time period so that the user interface screen includes the ECG signals (or part thereof), how the ECG signals are identified with respective types of arrhythmia, and optionally the statistics indicating the relative proportion of the types of arrhythmia associated with the signals for the selected prior time period.

In some exemplary embodiments, the apparatus receives signals output by electrodes (body surface electrodes and/or catheter electrodes) applied to a body of a human patient in response to electrical activity of a heart of the human patient. A set of templates corresponding to different types of arrhythmia of the heart are stored by the apparatus. The apparatus applies the templates to the ECG signals so as to associate one or more different temporal sections of the signals with respective types of arrhythmia, for example, by comparing the templates to the signals using image comparison techniques or any suitable signal comparison technique.

The apparatus computes a relative proportion (e.g., a percentage, fraction or ratio) of the types of arrhythmia associated with the signals. The relative proportion of an arrhythmia may be computed as the total time that the arrhythmia occurred out of the total time over which the ECG data under consideration was captured. The ECG data under consideration may be limited to the signals currently shown in the user interface screen or to some other configurable time window.

The apparatus renders the user interface screen including at least a part of the ECG signals, a time scale detailing the timing of the various parts of the signals, graphical identifiers identifying the different temporal sections of at least the part of the signals with the respective types of arrhythmia with which they are associated, and statistics indicating the relative proportion of the types of arrhythmia associated with the signals.

In one exemplary embodiment, the graphical identifiers may be color-coded identifiers using a different color for different types of arrhythmia. The graphical identifiers may be disposed in boxes within an information strip below or above the displayed signals. The statistics may be disposed in a color-coded legend which has a legend key for each arrhythmia type. The relative proportion statistic of an arrhythmia type may be disposed in the legend key for that arrhythmia type or at any other suitable position in the user interface screen.

In some exemplary embodiments, the apparatus may render another user interface screen including at least a part of another signal describing variations in a factor used in analysis of the heart. This user interface screen also includes graphical identifiers identifying different sections of at least the part of the other signal with different ranges of the variations in the factor, and statistics indicating a relative proportion of the different ranges of the variations in the factor. The factor may be selected from any one of the following by way of example only: a force applied by a probe, a tissue proximity to the probe, a velocity of the probe, a cycle length, a position stability of the probe, and a local activation time (LAT) stability.

For example, the signal may show how force applied by the probe varies over time. The graphical identifiers may identify different force ranges, e.g., 0-1 grams, 1-2 grams etc. The statistics may indicate the percentage of the force ranges, e.g., 20% at 0-1 grams, and 30% at 1-2 grams etc.

### SYSTEM DESCRIPTION

Documents incorporated by reference herein are to be considered an integral part of the application except that, to the extent that any terms are defined in these incorporated documents in a manner that conflicts with definitions made explicitly or implicitly in the present specification, only the definitions in the present specification should be considered.

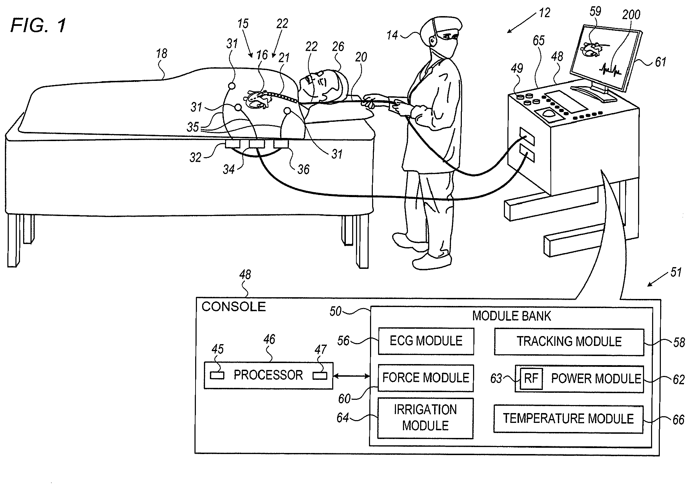

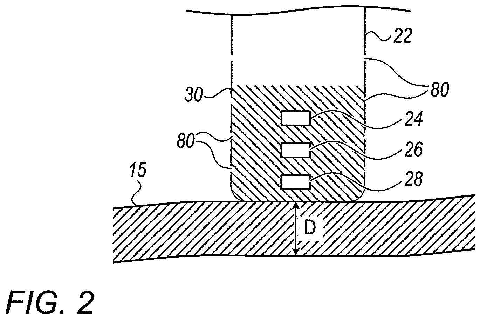

Reference is now made to Fig. 1, which is a schematic illustration of an invasive medical procedure using an apparatus 12 according to an exemplary embodiment of the present invention. Reference is also made to Fig. 2, which is a schematic illustration of a distal end 22 of a probe 20 used in the apparatus 12 according to an exemplary embodiment of the present invention. The procedure is performed by a physician 14, and in the description hereinbelow the procedure is assumed to comprise an ablation of a portion of tissue 15 of a myocardium 16 of the heart of a human patient 18.

In order to perform the investigation, the physician 14 inserts the probe 20 into a sheath 21 that has been pre-positioned in a lumen of the patient 18 so that the probe 20 is inserted into a chamber of the heart. The sheath 21 is positioned so that the distal end 22 of the probe 20 enters the heart of the patient 18. The distal end 22 comprises a position sensor 24 that enables the location and orientation of the distal end 22 to be tracked, a force sensor 26 that measures the force applied by the distal end 22 when it contacts the myocardium 16, and one or more temperature sensors 28 that measure the temperature at respective locations of the distal end 22. The distal end 22 also comprises one or more electrodes 30 which are used to apply radiofrequency power to the myocardium 16 in the chamber so as to ablate the myocardium 16. The electrode(s) 30 may also be used to acquire electropotentials from the myocardium 16, as noted below.

The apparatus 12 is controlled by a system processor 46, which is located in an operating console 48 of the apparatus. The operating console 48 comprises controls of at least one user input device 49 which are used by the physician 14 to communicate with the processor 46. The software for processor 46 may be downloaded to the processor 46 in electronic form, over a network, for example. Alternatively, or additionally, the software may be provided on non-transitory tangible media, such as optical, magnetic, or electronic storage media.

The processor 46 may comprise real-time noise reduction circuitry 45, typically configured as a field programmable gate array (FPGA), followed by an analog-to-digital (A/D) signal conversion integrated circuit 47. The processor 46 can pass the signal from the A/D signal conversion integrated circuit 47 to another processor and/or can be programmed to perform at least one algorithm disclosed herein, the algorithm comprising steps described hereinbelow. The processor 46 uses the noise reduction circuitry 45 and the A/D signal conversion integrated circuit 47, as well as features of modules which are described in more detail below, in order to perform the algorithm.

In order to operate the apparatus 12, the algorithm run by the processor 46 communicates with a module bank 50, which has a number of modules used by the processor 46 to operate the apparatus 12. Thus, the module bank 50 comprises an electrocardiograph (ECG) module 56 coupled to receive signals from body surface electrodes 31 and/or electrodes 30, in order to provide the ECG signals to the processor 46. The body surface electrodes 31 and/or the electrode(s) 30 are configured for application to a body of a subject (e.g., the patient 18) and configured to output signals in response to electrical activity of a heart of the subject. The electrode(s) 30 is applied to the heart of the body via the probe 20. The module bank 50 also includes a tracking module 58 which receives and analyzes signals from the position sensor 24, and which uses the signal analysis to generate a location and an orientation of the distal end 22. In some exemplary embodiments the position sensor 24 comprises one or more coils which provide the sensor signals in response to magnetic fields traversing the coils. In these exemplary embodiments, in addition to receiving and analyzing signals from sensor 24, tracking module 58 also controls radiators 32, 34, and 36 which radiate the magnetic fields traversing the position sensor 24. The radiators 32, 34, 36 are positioned in proximity to the myocardium 16, and are configured to radiate alternating magnetic fields into a region in proximity to the myocardium 16. A plurality of wire connections 35 link the operating console 48 with body the surface electrodes 31 and other components (such as the radiators 32, 34, 36 and the sensor 24) to enable the tracking module 58 to measure location and orientation coordinates of the probe 20. In some exemplary embodiments, the tracking module 58 is configured to compute a relative location and a relative orientation of the probe 20 with respect to the heart. Magnetic location and orientation tracking is described in U.S. Patent Nos. 7,756,576 and 7,536,218, which are hereby incorporated by reference. The Carto® system produced by Biosense Webster, of 33 Technology Drive, Irvine, CA 92618 USA, uses such a magnetic tracking system. The tracking module 58 is not limited to using magnetic based location and orientation tracking. Any suitable location and orientation tracking can be used, such as impedance-based or image-based tracking.

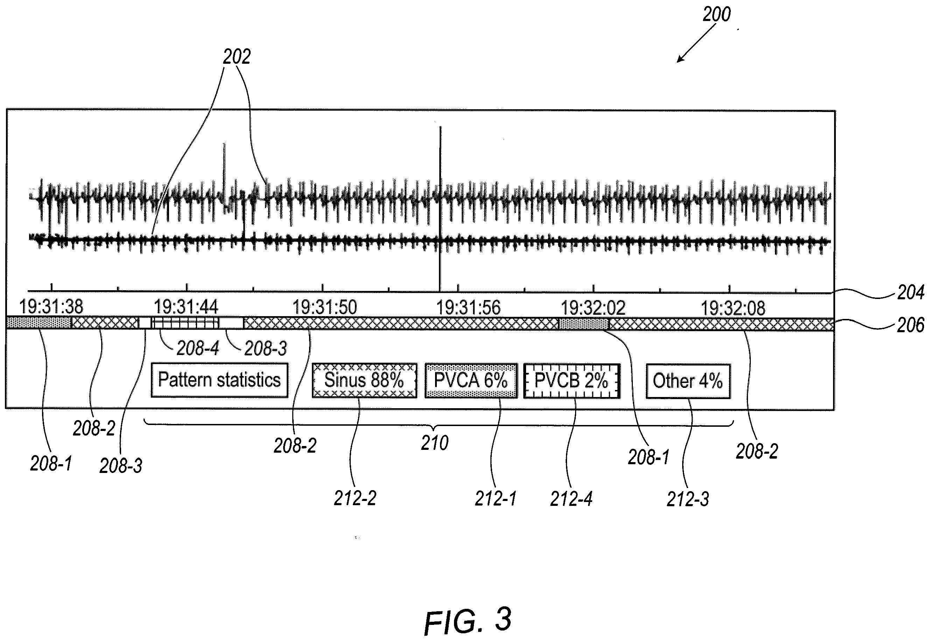

The apparatus 12 may receive image data from an external imaging modality, such as an MRI unit, CT unit or the like and includes image processors that can be incorporated in or invoked by the processor 46 for generating and displaying images. The image data may be registered with the tracking module 58 and an image 59 combining the received data and positions of the probe 20 may be displayed to the physician 14 on a display 61. For example, the track of the distal end 22 of the probe 20 may be shown on a three-dimensional representation of the heart of patient 18 that is displayed on the display 61. A user interface screen 200, described in more detail with reference to Fig. 3, may also be displayed on the display 61.

The electrode(s) 30 and the body surface electrodes 31 may be used to measure tissue impedance at the ablation site as taught in U.S. Patent No. 7,536,218, issued to Govari et al., which is herein incorporated by reference.

The module bank 50 also comprises a force module 60, a power module 62, an irrigation module 64, and a temperature module 66. The functions of these modules are explained below. The modules in the module bank 50, and the processor 46, are herein termed processing circuitry 51.

The force module 60 receives signals from the force sensor 26, and from the signals generates a magnitude of the contact force, herein assumed to be measured in grams, exerted by the distal end 22 on the tissue 15. In some exemplary embodiments the force sensor 26 is configured so that the signals it provides to the force module 60 enable the force module 60 to evaluate a direction of the force exerted by the distal end 22 on the tissue 15.

The power module 62 comprises a radiofrequency (RF) signal generator 63 which generates the radiofrequency power to be applied by the electrode(s) 30 to ablate the tissue 15 of the myocardium 16. The processor 46 and the power module 62 are able to adjust a power level, herein assumed to be measured in Watts, delivered by the electrode(s) 30, as well as a length of time, measured in seconds, during which the power is delivered.

The irrigation module 64 controls a rate of flow, herein assumed to be measured in mL/min, of irrigation fluid, typically normal saline solution, supplied to the distal end 22 by a pump 65 disposed in the operating console 48. The probe 20 includes an irrigation channel through which to irrigate the myocardium 16. The irrigation fluid is expelled from irrigation holes 80 in the distal end 22. The pump 65 is configured to selectively pump the irrigation fluid into the irrigation channel at an idle rate and at one or more one non-idle rates (higher than the idle rate) according to a status of the ablation procedure.

The temperature module 66 receives a temperature signal provided by the temperature sensor 28 (or by each temperature sensor 28). The temperature signal is indicative of a temperature of the myocardium at a plurality of different times. The temperature module 66 determines the temperatures registered by each of the sensors 28. Typically, in the case of multiple sensors 28 the temperature module 66 determines a mean temperature of the distal end 22. Additionally, in the case of multiple sensors, the temperature module 66 may produce a map of the temperature distribution of the distal end 22.

In some exemplary embodiments the electrocardiograph (ECG) module 56 may be implemented independently of the apparatus 12 without the probe 20 and the various ablation functionality described above. In these exemplary embodiments, the electrocardiograph (ECG) module 56 may be implemented in a stand-alone ECG system with a display screen for showing the user interface screen 200 or as part of any other suitable system.

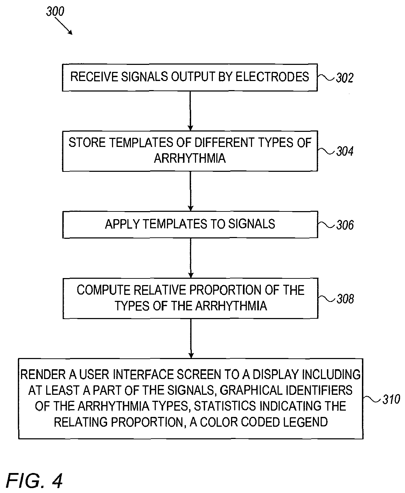

Reference is now made to Fig. 3, which is a view of the exemplary user interface screen 200 rendered by the apparatus 12 of Fig. 1. Reference is also made to Fig. 1. The body surface electrodes 31 and/or the electrode(s) 30 applied to the body of the human patient 18 output signals 202 in response to electrical activity of the heart of the human patient 18. The signals 202 may be processed by the processor 46 to remove noise. Other processing of the signals 202 is described in more detail with reference to Fig. 4 including identifying different arrhythmia types in the signals 202.

The processor 46 is configured to render to the display 61 the user interface screen 200, and example of which is shown in Fig. 3. The user interface screen 200 may include any one or more of the following:
- at least a part of the signals 202,
- a time scale 204 detailing the timing of the various parts of the signals 202,
- graphical identifiers 208 identifying the different temporal sections of at least the part of the signals 202 with the respective types of arrhythmia with which they (the sections) are associated,
- statistics indicating the relative proportion of the types of arrhythmia associated with the signals 202, and
- a color-coded legend 210.

The signals 202 may be scrolled (left and right) in the user interface screen 200 so that different temporal sections of the signals 202 may be shown in the user interface screen 200 at different times. As the signals 202 are scrolled the time scale 204 also scrolls corresponding to the timing associated with the signals 202 being shown. The graphical identifiers are also amended to reflect the types of arrhythmia included in the signals 202 now shown in the user interface screen 200.

In some exemplary embodiments, the graphical identifiers 208 are color-coded identifiers using a different color for the different types of arrhythmia. The graphical identifiers 208 are disposed in boxes within an information strip 206 below or above at least the part of the signals 202. The color-coded legend 210 includes a plurality of legend keys 212 where each key 212 textually describes its corresponding arrhythmia type and is colored according to the color indicating that arrhythmia type in the corresponding color-coded identifier. The statistics for each arrhythmia may be disposed in the legend key 212 for that arrhythmia type. Alternatively, the statistics may be disposed at any suitable position on the user interface screen 200. The statistics may be formatted as a percentage, fraction or ratio, by way of example only. The relative proportion of an arrhythmia may be computed as the total time that the arrhythmia occurred out of the total time over which the ECG data under consideration was captured. The total time used in the computation of the relative proportion may be the total time of the ECG signal currently shown on the user interface screen 200 or the total time of the ECG signal captured for a time period larger than the time period currently shown on the user interface screen 200 such as the time period for which ECG data is available or for a different time period configured by the physician 14.

In the example of Fig. 3, PVC-A arrhythmia is identified in the information strip 206 using the graphical identifier 208-1, in the color-coded legend 210 using the legend key 212-1, and its relative proportion of 6% in the legend key 212-1. Sinus arrhythmia is identified in the information strip 206 using the graphical identifier 208-2, in the color-coded legend 210 using the legend key 212-2, and its relative proportion of 88% in the legend key 212-2. PVC-B arrhythmia is identified in the information strip 206 using the graphical identifier 208-4, in the color-coded legend 210 using the legend key 212-4, and its relative proportion of 2% in the legend key 212-4. Other arrhythmia types are grouped and are identified in the information strip 206 using the graphical identifier 208-3, in the color-coded legend 210 using the legend key 212-3, and its relative proportion of 4% in the legend key 212-3.

In some exemplary embodiments, the color-coding of the graphical identifiers 208 may be replaced with, or supplemented by, different patterned identifiers and/or any suitable format for distinguishing the different arrhythmias including letters (e.g., PVC A, PVC B) and/or digits. In similar fashion, the color-coded legend 210 may be suitably amended to provide an appropriate legend for the graphical identifiers 208.

Additionally, or alternatively, the lines of the signals 202 may be colored or otherwise formatted to identify the different temporal sections of the signals 202 with the arrhythmia types.

Reference is now made to Fig. 4, which is a view of a flowchart 300 including exemplary steps in a method of operation of the apparatus 12 of Fig. 1.

The steps represented by blocks 302-310 may be performed in any suitable order. Additionally, some of the steps may be performed simultaneously.

The processor 46 is configured to receive (block 302) signals 202 (Fig. 3) output by the body surface electrodes 31 and/or the electrode(s) 30 applied to the body of the human patient 18 in response to electrical activity of the heart of the human patient 18. The processor 46 is configured to store (block 304) a set of templates corresponding to different types of arrhythmia of the heart.

The processor 46 is configured to apply (block 306) the templates to the signals 202 so as to associate one or more different temporal sections of the signals 202 with respective ones of the types of arrhythmia. This step may include comparing the templates to the signals 202, for example, using image comparison techniques or any suitable signal comparison technique.

The processor 46 may be configured to compute (block 308) a relative proportion of the types of arrhythmia associated with the signals 202 as a percentage, fraction or ratio, by way of example only. The relative proportion of an arrhythmia type may be computed as the total time that the arrhythmia type occurred out of the total time over which the ECG data under consideration was captured. The total time used in the computation of the relative proportion may be the total time of the ECG signal 202 currently shown on the user interface screen 200 or the total time of the ECG signal captured for a time period larger than the time period currently shown on the user interface screen 200 such as the time period for which ECG data is available or for a different time period configured by the physician 14.

The processor 46 is configured to render (block 310) the user interface screen 200 to the display 61. The user interface screen 200 may include any one or more of the following:
- at least a part of the signals 202,
- a time scale 204 detailing the timing of the various parts of the signals 202,
- graphical identifiers 208 identifying different temporal sections of at least the part of the signals 202 with the respective types of arrhythmia with which the sections are associated,
- statistics indicating the relative proportion of the types of arrhythmia associated with the signals 202, and
- a (color-coded) legend 210.

In accordance with some exemplary embodiments, the processor 46 is configured to provide data of one or more other factors that may be selected from any one of the following: a force applied by the probe 20, a tissue proximity to the probe 20, a velocity of the probe 20, a cycle length, a position stability of the probe 20, and a local activation time (LAT) stability. The processor 46 is configured to compute statistics for a selected factor as described below in more detail. The processor 46 is configured to render to the display 61 another user interface screen substantially the same as the user interface screen 200 but with modifications relevant to the selected factor. This other user interface screen may include:
- at least a part of another signal(s) describing variations in the selected factor used in analysis of the heart,
- a time scale detailing the timing of the various parts of the other signal(s) of the selected factor,
- graphical identifiers identifying different sections of at least the part of the other signal(s) of the selected factor with different ranges of the variations in the selected factor,
- statistics indicating a relative proportion of the different ranges of the variations in the selected factor, and
- a (color-coded) legend.

By way of example, when force is the selected factor, the signal is a representation of force applied by the probe 20 versus time, and the different ranges of the factor may be 0-1 grams, 1-2 grams etc. In this example, the statistics may be 30% for 0-1 grams and 20% for 1-2 grams etc.

By way of example, when tissue proximity to the probe 20 is the selected factor, the signal is a representation of tissue proximity versus time, and the different ranges of the factor may be 0-1 mm, 1-2 mm etc. In this example, the statistics may be 10% for 0-1 mm and 20% for 1-2 mm etc.

By way of example, when velocity of the probe 20 is the selected factor, the signal is a representation of velocity versus time, and the different ranges of the factor may be 0-0.01 meters per second (m/s), 0.01-0.02 m/s etc. In this example, the statistics may be 10% for 0-0.01 m/s and 15% for 0.01-0.02 m/s etc.

By way of example, when cycle length is the selected factor, the signal is a representation of cycle length versus time, and the different ranges of the factor may be 0-50 milliseconds (ms), 50-100 ms etc. In this example, the statistics may be 50% for 0-50 ms and 10% for 50-100 ms etc.

By way of example, when position stability of the probe 20 is the selected factor, the signal is a representation of position stability versus time, and the different ranges of the factor may be 0-1 mm, 1-2 mm etc. In this example, the statistics may be 10% for 0-1 mm and 20% for 1-2 mm etc.

By way of example, when local activation time (LAT) stability is the selected factor, the signal is a representation of LAT stability versus time, and the different ranges of the factor may be 0-10 ms, 10-20 ms etc. In this example, the statistics may be 23% for 0-10 ms and 45% for 1-2 ms etc.

In some exemplary embodiments in which the other user interface screen is rendered, the graphical identifiers are color-coded identifiers using a different color for the different ranges of the selected factor. The graphical identifiers may be disposed in boxes within an information strip below or above at least the part of the signal(s). The color-coded legend may include legend keys where each key textually describes its corresponding factor range and is colored according to the color indicating that factor range in the corresponding color-coded identifier. The statistics for each factor range may be disposed in the legend key for that factor range or in any other suitable position in the other user interface screen. The statistics may be formatted as a percentage, fraction or ratio, by way of example only. The relative proportion of a factor range may be computed as the total time that the factor range occurred out of the total time over which the factor data under consideration was captured. The total time used in the computation of the relative proportion may be the total time of the factor signal currently shown on the other user interface screen or the total time for a time period larger than the time period currently shown on the other user interface screen such as the time period for which factor signal data is available or for a different time period configured by the physician 14. In some exemplary embodiments, the color-coding of the graphical identifiers may be replaced with, or supplemented by, different patterned identifiers and/or any suitable format for distinguishing the different factor ranges including letters (e.g., Force range A, Force range B) and/or digits. In similar fashion, the color-coded legend may be suitably amended to provide an appropriate legend for the graphical identifiers. Additionally, or alternatively, in rendering the other user interface screen, the lines of the signal(s) may be colored or otherwise formatted to identify the different temporal sections of the signal(s) with the different factor ranges.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The exemplary embodiments described above are cited by way of example, and the present invention is not limited by what has been particularly shown and described hereinabove. Rather the scope of the invention includes both combinations and subcombinations of the various features described hereinabove, as well as variations and modifications thereof which would occur to persons skilled in the art upon reading the foregoing description and which are not disclosed in the prior art.

## Claims

1. A system for automatically identifying and displaying arrhythmias, the system comprising:
electrodes configured for application to a body of a subject and configured to output signals in response to electrical activity of a heart of the subject;
a display; and
a processor configured to:
store a set of templates corresponding to different types of arrhythmia of the heart;
apply the templates to the signals so as to associate one or more different temporal sections of the signals with respective ones of the types of arrhythmia; and
render to the display a user interface screen including at least a part of the signals and graphical identifiers identifying the different temporal sections of at least the part of the signals with the respective ones of the types of arrhythmia with which they are associated.

2. The system according to claim 1, wherein processor is configured to: compute a relative proportion of the types of arrhythmia associated with the signals; and render to the display the user interface screen including at least the part of the signals, the graphical identifiers, and statistics indicating the relative proportion of the types of arrhythmia associated with the signals.

3. The system according to claim 1, wherein processor is configured to render to the display another user interface screen including at least a part of another signal describing variations in a factor used in analysis of the heart, and graphical identifiers identifying different sections of at least the part of the other signal with different ranges of the variations in the factor, the factor being selected from any one of the following: a force applied by a probe, a tissue proximity to the probe, a velocity of the probe, a cycle length, a position stability of the probe, and a local activation time (LAT) stability.

4. The system according to claim 3, wherein the processor is configured to generate the other user interface screen also to include statistics indicating a relative proportion of the different ranges of the variations in the factor.

5. A method for automatically identifying and displaying arrhythmias, the method comprising:
receiving signals output by electrodes applied to a body of a subject in response to electrical activity of a heart of the subject;
storing a set of templates corresponding to different types of arrhythmia of the heart;
applying the templates to the signals so as to associate one or more different temporal sections of the signals with respective ones of the types of arrhythmia; and
rendering to a display a user interface screen including at least a part of the signals and graphical identifiers identifying the different temporal sections of at least the part of the signals with the respective ones of the types of arrhythmia with which they are associated.

6. The system according to claim 1 or the method according to claim 5, wherein the graphical identifiers are color-coded identifiers using a different color for different ones of the types of arrhythmia.

7. The system or method according to claim 6, wherein the graphical identifiers are disposed in boxes below or above at least the part of the signals.

8. The system according to claim 6, wherein processor is configured to: compute a relative proportion of the types of arrhythmia associated with the signals; and render to the display the user interface screen including at least the part of the signals, the graphical identifiers, and statistics indicating the relative proportion of the types of arrhythmia associated with the signals.

9. The system according to claim 8, wherein the statistics are disposed in a color-coded legend having a plurality of legend keys, each legend key textually describing one of the types of arrhythmia corresponding with one of the color-coded identifiers.

10. The method according to claim 6, further comprising computing a relative proportion of the types of arrhythmia associated with the signals, wherein the rendering includes rendering to the display the user interface screen including at least the part of the signals, the graphical identifiers, and statistics indicating the relative proportion of the types of arrhythmia associated with the signals.

11. The method according to claim 10, wherein the statistics are disposed in a color-coded legend having a plurality of legend keys, each legend key textually describing one of the types of arrhythmia corresponding with one of the color-coded identifiers.

12. The method according to claim 5, further comprising computing a relative proportion of the types of arrhythmia associated with the signals, wherein the rendering includes rendering to the display the user interface screen including at least the part of the signals, the graphical identifiers, and statistics indicating the relative proportion of the types of arrhythmia associated with the signals.

13. The method according to claim 5, further comprising rendering to the display another user interface screen including at least a part of another signal describing variations in a factor used in analysis of the heart, and graphical identifiers identifying different sections of at least the part of the other signal with different ranges of the variations in the factor, the factor being selected from any one of the following: a force applied by a probe, a tissue proximity to the probe, a velocity of the probe, a cycle length, a position stability of the probe, and a local activation time (LAT) stability.

14. The method according to claim 13, further comprising generating the other user interface screen also to include statistics indicating a relative proportion of the different ranges of the variations in the factor.

15. A software product for automatically identifying and displaying arrhythmias, comprising a non-transient computer-readable medium in which program instructions are stored, which instructions, when read by a central processing unit (CPU), cause the CPU to:
receive signals output by electrodes applied to a body of a subject in response to electrical activity of a heart of the subject;
store a set of templates corresponding to different types of arrhythmia of the heart;
apply the templates to the signals so as to associate one or more different temporal sections of the signals with respective ones of the types of arrhythmia; and
render to a display a user interface screen including at least a part of the signals and graphical identifiers identifying the different temporal sections of at least the part of the signals with the respective ones of the types of arrhythmia with which they are associated.
